(19) Europäisches Patentamt
European Patent Office
Office européen des brevets

(11) EP 1 371 368 A1

(12) EUROPEAN PATENT APPLICATION

(43) Date of publication:
**17.12.2003 Bulletin 2003/51**

(21) Application number: **02077271.1**

(22) Date of filing: **11.06.2002**

(51) Int Cl.[7]: **A61K 31/216**, A61K 31/192, A61K 31/343, A61K 35/78, A61P 3/04

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **N.V. Nutricia**
**2700 MA Zoetermeer (NL)**

(72) Inventors:
- **De Bont, Hendricus Bartholomeas Andreas**
  **6721 EH Bennekom (NL)**
- **Raggers, Rene John**
  **1057 NX Amsterdam (NL)**

(74) Representative: **van Westenbrugge, Andries et al**
**Nederlandsch Octrooibureau**
**P.O. Box 29720**
**2502 LS The Hague (NL)**

# (54) Salvianolic acid components as lipase inhibitors

(57) The present invention relates to a method for the treatment and/or prevention of overweight and/or related disorders, comprising administering to a mammal an effective amount of salvianolic acid component (SA component), a pharmaceutically acceptable salt or a precursor thereof, wherein the SA component has the formula

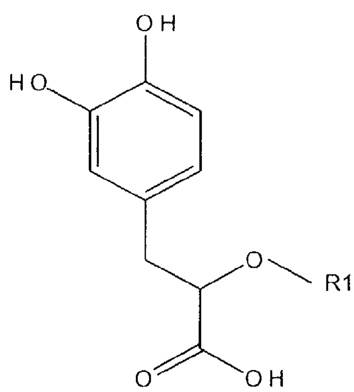

in which R1 is selected from $C_{1-50}$ hydrocarbyl, $C_{1-50}$ substituted hydrocarbyl, $C_{1-50}$ heterohydrocarbyl, $C_{1-50}$ substituted heterohydrocarbyl.

The invention also relates the use of the SA component as a lipase inhibitor as well as to compositions, including food products, containing the SA component.

EP 1 371 368 A1

## Description

### FIELD OF THE INVENTION

The present invention relates to a method for the treatment and/or prevention of overweight and related disorders in a mammal, said method comprising the administration of a component capable of preventing fat absorption.

### BACKGROUND OF THE INVENTION

Prevention of body weight increase can be accomplished by inhibiting or stimulating a variety of mechanisms. For example, lipolysis of triglycerides stored within the adipocyte can be stimulated (e.g. with caffeine), appetite can be reduced (e.g. with hydroxycitric acid or fenfluramine) and triglyceride absorption can be inhibited (e.g. with Orlistat).

An effective strategy of combating obesity is reducing the intestinal absorption of ingested triglycerides, for example by inhibiting gastrointestinal lipase. A widely used antiobesic agent capable of inhibiting gastrointestinal lipase is Orlistat, previously known as tetrahydrolipstatin or THL. Orlistat reduces the absorption of dietary fat. Its use for the control or prevention of obesity and hyperlipidaemia is described in U.S. Pat. No. 4,598,089. However, many people are reluctant to use Orlistat, since it is expensive and a non-natural chemical.

Additionally, herbal preparations have been described to reduce lipase activity. JP10262606 describes that plants of the family Labiatae are capable of controlling accumulation of lipid *in vivo,* which consequently contributes to suppression and the prevention of obesity. The family Labiatae comprises at least about 5000 plant species. The document only describes a few specific plant species of the family Labiatae or extracts thereof for use as a lipase inhibitor, i.e. rosemary, Isodon japanica, Isodon trichocarpus, Isodon sikokianus var. intermodius and ennmeisou (grass of Plectranthus rugosus).

Presently, many herbal preparations are used in methods for the treatment of obesity, while the active ingredient(s) responsible for the weight reducing effects are unknown. Without the active ingredient(s) identified, the efficacy of the plant material cannot be predicted. The result is that people, who have the desire to decrease their body weight, may ingest plant material that does not have an optimal effect, e.g. a plant extract that contains only an ineffective amount of the active ingredient. Additionally, without the active ingredient having been identified, it is unclear which weight reducing mechanism is triggered following the ingestion of the plant extract. This may result in an ineffective administration regimen. For instance, ingestion of a lipase inhibitor about 4 hours before a fat containing meal generally provides a very limited weight reducing effect.

Plant material from Salvia species and active constituents thereof are known to have a variety of beneficial effects on health. Plant extracts of Salvia species are for example used to promote healthy hair growth (US6183749); to enhance cardiovascular function (US6299910); to treat hepatic disorders caused by a hepatitis C virus infection (US6126942); and to inhibit *in vitro* HIV infection in human T lymphocyte cells and mononuclear phagocytic lineage cells infected with HIV (US5178865).

Additionally, Salvia plant material has been used in nutritional supplements advertised to reduce body weight. An example of a nutritional supplement advertised to reduce weight and lower blood lipids is Slim)ex® (Blue Sky Green Earth, South Africa).

Salvia species contain a large variety of organic molecules, which have been shown to provide a beneficial effect on health, such as tanshinones, rosemaric acid, lithospermic acids, salvianolic acids and caffeic acid. Salvianolic acids have been described to posses antiviral activity, antiulcer effects, and hydroxyl radical scavenging effects. E.g. salvianolic acid B has been described to protect the brain against injuries caused by ischemia-reperfusion and to inhibit fibril formation and neurotoxicity of amyloid beta-protein in vitro. Salvianolic acid A has been described to have protective effects against impairment of memory induced by cerebral ischemia-reperfusion in mice, to have a protective effect on ischemia-reperfusion-induced injury in the isolated rat heart and inhibit cataract.

### SUMMARY OF THE INVENTION

In order for the body to make use of dietary fat, the dietary fat must first be absorbed from the small intestine. Since the dietary fat is mostly ingested in the form of oils, fats or fat continuous products, these first have to be emulsified. The emulsification of dietary lipids is accomplished by means of bile salts, which are synthesized from cholesterol in the liver, stored in the gallbladder and secreted following the ingestion of fat. The emulsification of dietary fats by bile acids renders them accessible to pancreatic lipases. These lipases hydrolyze the dietary fats, generating free fatty acids and a mixture of mono- and diacylglycerols. Pancreatic lipase degrades triacylglycerols at the 1 and 3 positions sequentially to generate 1,2-diacylglycerols, 2,3-diacylglycerols and 2-acylglycerols. These products then diffuse into or are transported into the intestinal epithelial cells, where the re-synthesis to triacyglycerols occurs.

Lipase inhibitors reduce the action of lipases, thereby reducing the rate of the conversion of dietary triacylglycerols (dietary fats) to 1,2-diacylglycerols, 2,3-diacylglycerols, 2-acylglycerols and fatty acids. As a result, the triacylglycerols remain in the intestinal tract and will not be absorbed. The unabsorbed triacylglycerols are subsequently transported to the lower parts of the intestinal tract and are either excreted or fermented by the intestinal flora. The inhibition of intestinal lipase thus results in a reduced intestinal absorption of the (high caloric) dietary fats.

The present inventors have surprisingly found that salvianolic acid component (SA component) is capable of inhibiting the action of pancreatic lipase. SA component is thus capable of inhibiting the intestinal absorption of dietary fats from ingested food or feed. Enteral administration of SA component therefore results in a decreased absorption of high caloric ingredients from the diet. Hence, the present invention provides a method for the treatment or prevention of overweight and related disorders in mammals, comprising the administration of an effective amount of SA component.

The finding that SA component is capable of reducing intestinal lipase activity enables the manufacture of weight reducing compositions from SA component containing plants or plant parts, wherein the weight reducing efficacy of the (herbal) composition can be predicted based on its the chemical composition (i.e. SA component content) and/or standardized.

Until now, the weight reducing effect of SA component was unknown. Hence, known products for the treatment of obesity that contain Salvia plant material have not been optimized to take full advantage of the lipase inhibiting effect of the SA component. Indeed, such commercial products have been found to contain an ineffective amount of SA component.

In further aspect, the present invention provides novel compositions containing SA component and at least one ingredient selected from the group consisting of plant fibers capable of reducing a fatty stool, chitosan and fat-soluble vitamins. These compositions can be advantageously used in a method for reducing overweight, without side effects such as fatty stool or reduced absorption of fat-soluble vitamins.

DETAILED DESCRIPTION OF THE PREFFERED EMBODIMENTS

*Chemical structure*

The present invention relates to a method for the treatment and/or prevention of overweight and/or related disorders, comprising administering to a mammal an effective amount of salvianolic acid component (SA component), a pharmaceutically acceptable salt or a precursor thereof, wherein the SA component has the formula

*Formula I*

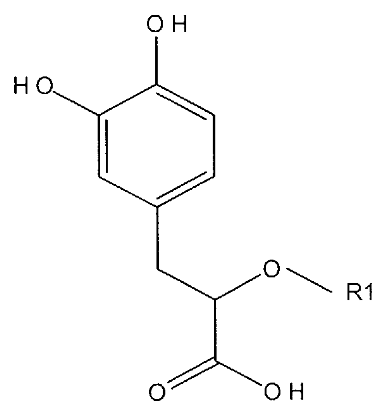

in which R1 is selected from $C_{1-50}$ hydrocarbyl, $C_{1-50}$ substituted hydrocarbyl, $C_{1-50}$ heterohydrocarbyl, $C_{1-50}$ substituted heterohydrocarbyl.

Preferably R1 represents (CO)-R2 in which R2 is selected from optionally substituted alkyl, alkenyl, alkynyl, cycloalkyl, heterocyclyl, aryl and heteroaryl.

Preferably R2 represents CH=CH-R3, in which R3 is selected from optionally substituted alkyl, alkenyl, alkynyl, cycloalkyl, heterocyclyl, aryl and heteroaryl.

Preferably R3 represents an optionally substituted hydroxy-aryl. More preferably R3 has the following formula

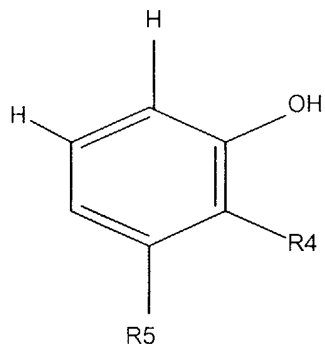

in which:

(i) R4 represents hydroxyl; R5 is selected from optionally substituted alkyl, alkenyl, alkynyl, cycloalkyl, heterocyclyl, aryl and heteroaryl; or
(ii) R4 and R5 together form an optionally substituted tetrahydrofuran radical in which R4 represents O-.

The term precursor as used in the present invention refers to those compounds, which can be converted to the SA component according to the present invention in the mammalian intestinal tract.

Even more preferably the SA component has one of the following structures (formula II, formula III, formula IV or formula V):

*Formula II*

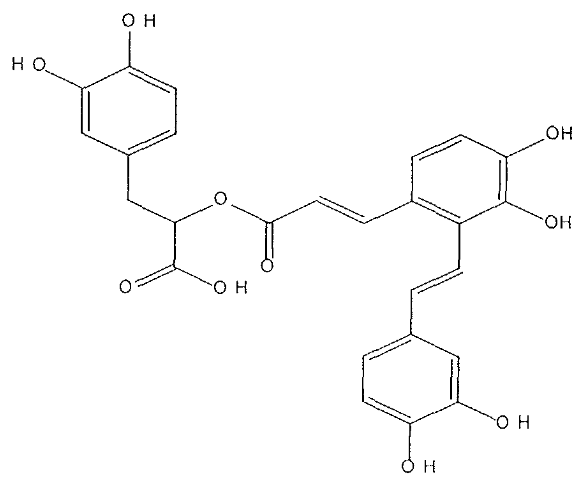

or

*Formula III*

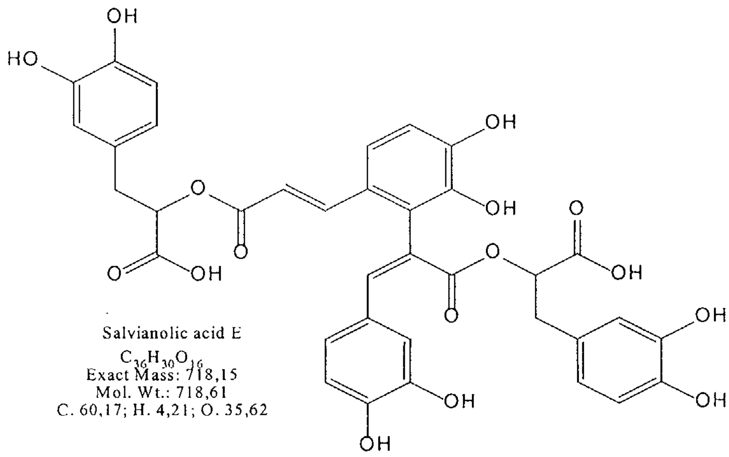

Salvianolic acid E

$C_{36}H_{30}O_{16}$
Exact Mass: 718,15
Mol. Wt.: 718,61
C. 60,17; H. 4,21; O. 35,62

or

*Formula IV*

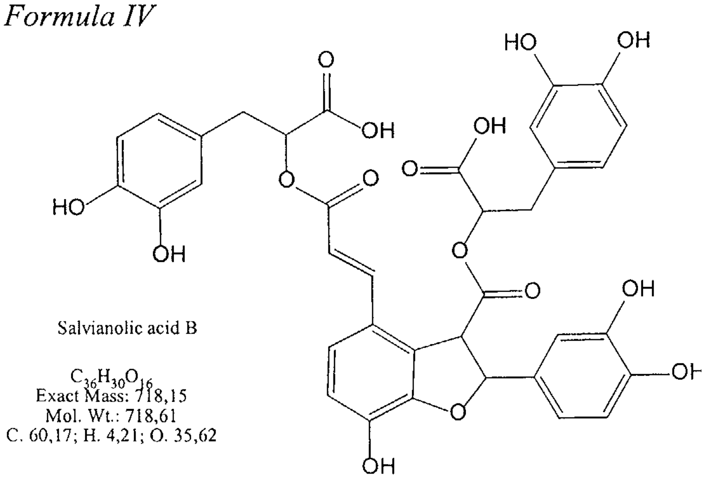

Salvianolic acid B

$C_{36}H_{30}O_{16}$
Exact Mass: 718,15
Mol. Wt.: 718,61
C. 60,17; H. 4,21; O. 35,62

or

*Formula V*

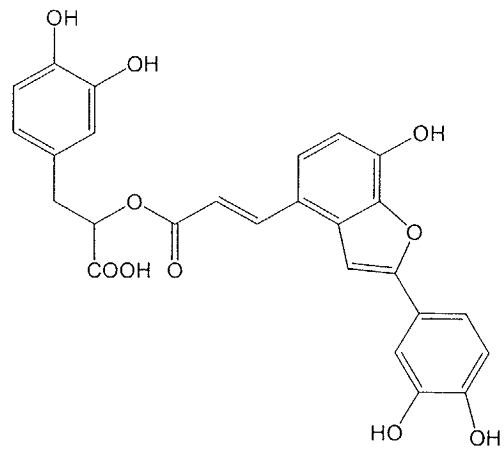

*General formula*

Preferably the chemical formula of the salvianolic acid used in the present method is $C_xH_yO_z$ wherein:

x has a value between 10 and 100, preferably between 15 and 50; and
y has a value between 10 and 150, preferably between 15 and 50; and
z has a value between 5 and 50, preferably between 6 and 25.

The SA component may also be provided in the form of an edible salt of any one of the above described structures or chemical formulas. Suitable salts of the SA component include potassium, calcium, magnesium and sodium salts
Preferably the SA component includes at least at least two dihydroxyphenyl moieties, even more preferably at least 3.
The invention encompasses monomers, dimers, trimers and polymers of SA component, which are covalently linked and possess the capacity to reduce lipase activity.
According to a further preferred embodiment the SA component used in the present method is a naturally occurring salvianolic acid. Suitable naturally derivable SA components can be selected from the group consisting of salvianolic acid A, B, C, I, D, E, F, J, K, salts of any one of the preceding salvianolic acids, precursors of any one of the preceding salvianolic acids or salts and mixtures thereof. According to a particularly preferred embodiment the SA component is selected from the group consisting of salvianolic acid A, B, C E, salts thereof, precursors thereof and mixtures of these salvianolic acids, salts and/or precursors.

*Sources of salvianolic acid*

The SA component used in the present method may be chemically manufactured (e.g. from oleochemicals), biochemically produced (e.g. in fermentation processes), or be obtained from vegetable material, optionally followed by subsequently chemical modification. The SA component used in the present method may for example be (bio) chemically manufactured by esterifying 3-(3,4-dihydroxyphenyl)lactic acid with a carboxylic acid.
The SA component is preferably isolated from plant material, particularly from the roots of plants. Preferred plant sources of the SA component are plants belonging to the genus Salvia, particularly *Salvia miltiorrhiza, Salvia cavaleriei, Salvia fluva, Salvia chinensis, Salvia bowleyana, Salvia prionitis, Salvia officialis, Salvia deserta* and/or *Salvia yunnanensis.* Most preferably, the SA component is obtained from *Salvia milthiorrhiza.*
In a preferred embodiment, the present method comprises the administration of an effective amount of a plant isolate from one of the above-mentioned Salvia species, said plant isolate containing least 5 wt.% SA component, based on dry weight of the plant isolate, preferably at least 10 wt.%, even more preferably at least 25 wt.%, most preferably at least 40 wt.%. The plant isolate is preferably prepared by solvent extraction, more preferably by extraction of the plant raw material with an aqueous solvent having a pH above 7. According to an even more preferred embodiment, the plant isolate is prepared by an isolation process which includes at least a solvent extraction and at least a

chromatographic separation technique.

Methods for the production of plant isolates containing at least 10 wt.% SA component are known in the art, for example from Xu et al, 2001 (US6299910).

*Dosages of salvianolic acid*

The present method comprises the administration of an effective amount of salvianolic acid. Preferably, a daily amount of between 0.1 and 250 mg SA component per kg body weight is administered to a mammal, more preferably between 0.5 and 100 mg/kg body weight, even more preferably between 1 and 50 mg/kg body weight, most preferably between 2.5 and 20 mg/kg body weight.

*Treatment*

The present invention provides a method for reducing or preventing overweight and related disorders, i.e. a method for the prophylactic or curative treatment of overweight and related disorders.
The term overweight as used in the present invention refers to a bodyweight that is above the desired bodyweight of a human subject or that of a pet or farm animal as defined by its owner. Human subjects who have a body mass index above 25 most advantageously use the present method.

The SA component is effective in reducing body weight since it has the capacity to inhibit the activity of intestinal lipase, and thereby reduce the fat absorption. The term "fat absorption" refers to the reduced intestinal absorption of enterally administered triglycerides or degradation products of enterally administered triglycerides. The present method can thus be advantageously used for the inhibition of intestinal lipase activity and for the reduction of intestinal dietary fat absorption. The reduced fat absorption in the intestinal tract results in a decrease of triglycerides and other fats in the blood. The present invention therefore also provides a method for the prophylactic and/or curative treatment of obesity related disorders, particularly hyperlipdemia and hypercholesterolemia.

Absorbed dietary fats are usually rapidly converted to triglycerides and stored in adipose tissue. Reduction of dietary fat absorption with the present method results in reduced formation of adipose tissue mass compared to a situation wherein the SA component is not administered. The present method can thus suitably be used to reduce adipose tissue mass or prevent an increase in adipose tissue mass, a method for increasing the ratio lean body mass to adipose tissue mass and/or the ratio muscle mass to adipose tissue mass.

*Administration*

Preferably the SA component is incorporated in a pharmaceutical preparation or nutritional supplement. Packaged nutritional supplements and dietary products, which have been provided with labels that explicitly or implicitly direct the consumer towards the use of said supplement or product in accordance with one or more of the above or below purposes (e.g. reduction of overweight, reduction of adipose tissue mass), are encompassed by the present invention.

In accordance with the present method the SA component is administered enterally. Preferably the SA component is administered orally. The SA component used in the present method can be applied in any suitable form, such as meals, bars, pills, capsules, gels, biscuits, drinks, yellow fat spreads etc.

According to a further preferred embodiment the SA component is provided in a unit dosage form. The term unit dosage form refers to a physically discrete unit suitable for unitary administration to human subjects and other mammals, wherein each unit contains a predetermined quantity of salvianolic acid and a pharmaceutically acceptable carrier.

The aforementioned unit dosage form is preferably in a solid or semisolid form, more preferably in the form of an oral dosage unit, which term includes capsules, tablets, microparticles and microspheres.

The SA component used in the present method may be modified in environments of the upper intestinal tract and is therefore preferably orally administered in a unit dosage form that is coated with a substance that can withstand the enteric environment (an enteric coating) to prevent the decrease of its lipase inhibitory activity.

The solid or semisolid unit dosage form preferably has a weight between 0.1 and 30 grams, more preferably between 0.2 and 10 gram. When an oral dosage unit is used to provide the SA component, it preferably has a weight between 0.2 and 4 grams, even more preferably between 0.5 and 3 grams. In the present method a daily dosage of SA component can include one or more unit dosage forms, preferably the daily dosage consists of 1 to 6 unit dosage forms.

In the present method, the SA component is preferably administered between 60 minutes before and 60 minutes after the ingestion of a significant amount of triglycerides, e.g. at least 2 grams of triglycerides. According to a further preferred embodiment, the SA component is ingested prior to, during or shortly after a meal.

*SA component in foods*

The SA component may also be advantageously incorporated in fat containing foods or feeds. In this way, the consumer can enjoy the consumption of the fat containing product, but with reduced impact on weight gain. Hence, the present invention also provides a fat containing food or feed, comprising a lipase inhibitor, particularly SA component. The term fat containing food or feed refers to those food and/or feed products which contain between 10 wt.% and 95 wt.%, particularly between 25 wt.% and 90 wt.% triglycerides based on the total weight of the food product. The inclusion of SA component in the fat containing food product is particularly advantageous in those food products that are usually consumed in significant quantities, i.e. typically at least 5 gram within 1 hour. The food product preferably contains between 0.01 mg and 500 mg SA component per gram triglyceride, more preferably between 0.1 and 50 mg SA component per gram triglycerides. Preferably the triglyceride containing food or feed comprises between 0.01 and 10 wt.% SA component based on the total weight of the food or feed. A preferred embodiment of the present invention provides spread, oils, shortenings, margarine, mayonnaise and dressings containing an effective amount of SA component. In a particular embodiment, the present invention provides a yellow fat spread containing an effective amount of SA component.

*Anal leakage*

Anal leakage of oil is an adverse effect, which is occasionally observed in patients treated with lipase inhibitors. It results from physical separation of some liquid, unabsorbed dietary fat from the bulk of the fecal mass in the lower large intestine. This effect can be prevented and/or treated with fat binding polymers, particularly chitosan and/or fat binding plant fibers. The present invention provides a novel composition comprising SA component and fat binding polymers. This novel composition can be suitably used in the present method. Fat binding polymers are polymers, which absorb, bind or otherwise associate liquid unabsorbed dietary fat and prevents the physical separation of the dietary fats from the fecal bulk. In the present method, SA component and fat binding polymer are preferably simultaneously administered, although sequential administration within 1 hour also is acceptable. The composition preferably contains at least between 1 and 100 mg fat binding polymer per mg SA components, more preferably between 2 and 50 mg fat binding polymer per mg SA component.

Preferably the coadministered fat binding polymer is an insoluble crude fiber and/or a food grade thickener or emulsifier. The term food grade thickener or emulsifier encompasses any conventional thickener or emulsifier used in foods. Preferred thickeners for use with the present invention are water-soluble natural, synthetic or semisynthetic polysaccharides, such as methylcellulose, xanthan gum, psyllium seed, ispaghula husk, plantago ovata seeds, karaya gum and mixtures of such compounds. Examples of suitable crude fibers are microcrystalline cellulose, for example, AVICEL®, wheat bran and oat bran. Preferably the fat binding polymer is selected from the group consisting of plant fiber capable of reducing fatty stool, chitosan and mixtures thereof

The fat binding polymer is preferably administered with SA component in a daily amount between 0.5 and 150 mg/kg body weight, more preferably between 1 and 100 mg/kg body weight. Suitable compositions for reducing or preventing overweight and related disorders contain between 25 mg and 10 gram SA component and between 50 mg and 50 g plant fiber capable of reducing fatty stool and/or chitosan, preferably between 250 mg and 25 grams of a plant fiber capable of reducing fatty stool and/or chitosan.

*Fat soluble vitamins*

To prevent a deficiency in fat-soluble vitamins, fat-soluble vitamins are preferably administered to the mammal during the period wherein SA component is ingested. Thus, in the present method, fat-soluble vitamins are advantageously (co)administered with the SA component. The present invention also provides novel compositions, preferably unit dosages, which contain SA component and fat-soluble vitamins.

Preferably one or more vitamins selected from the group consisting of vitamin A, vitamin D, vitamin E and vitamin K are included in a composition containing SA component. In the present method, the SA component and fat-soluble vitamins are preferably simultaneously administered, although sequential administration is also acceptable. Preferably, the novel composition, which can be advantageously used as a unit dosage in the present method, contains between 10% and 250% of the RDA of one or more of the above mentioned fat-soluble vitamins, more preferably between 25 and 125 %. The RDA's of each vitamin has been published by the US Food and Drug Administration.

*Calcium*

In a further preferred embodiment the present method comprises the administration of calcium, e.g. in the form of a calcium salt. Calcium will bind the fatty acids resulting from the hydrolysis of intestinal lipase activity, reducing

the quantity of fatty acid which are transported into the intestinal epithelial cells. Hence, coadministration of calcium in the present method will further reduce the fat absorption and contribute to the reduction and/or prevention of overweight. Preferably calcium is administered in an amount between 0.5 and 100 mg calcium per kg body weight on a daily bases, more preferably in an amount between 1 and 15 mg/kg body weight. Suitable compositions for reducing or preventing overweight and related disorders contain between 25 mg and 10 gram SA component and between 35 mg and 5 g calcium, preferably between 100 mg and 2 g calcium.

*Use of salvianolic acid for inhibiting lipase activity*

Lipase inhibitors can be advantageously used in several industrial processes. Hence the present invention provides the use of SA component for the inhibition of lipase. In a preferred embodiment the SA component is used to inhibit lipase activity in enzyme mixtures used in the manufacture of protein hydrolysate. During the manufacture of protein hydrolysate, enzyme mixtures containing mainly proteases, are incubated with the protein mix. The presence of even minor amounts of lipases in the protease containing enzyme mix will result in the hydrolysis of triglycerides and the formation of fatty acids having an undesirable cheesy taste and/or smell. The SA component can thus be advantageously used to prevent the hydrolysis of triglycerides in the manufacture of protein hydrolysate.

## EXAMPLES

Example 1: *In vitro* lipase inhibitory activity of salvianolic acids containing material:

Lipase activity was determined by measuring the rate of hydrolysis of p-nitrophenyl caprylate to p-nitrophenol (yellow) and caprylate. In order to assess the potency of the lipase inhibitor, the concentration of the lipase inhibitor, which resulted in a 50% inhibition of lipase activity (compared to control) was determined and compared to the lipase inhibiting activity of a known lipase inhibitor (Orlisat).

*Preparation of solutions*

Phosphate buffer A (200 mM phosphate buffer with a pH 7) was prepared by mixing: 488 ml 200 mM $Na_2HPO_4{\cdot}H_2O$ (17.5 gram/500 ml) with 296 ml 200 mM $NaH_2PO_4{\cdot}H_2O$ (13.8 gram/500 ml) followed by adjustment of the pH.
Substrate solution was prepared freshly by mixing:

a) 500 ml buffer B (200 mM phosphate buffer A pH 7 containing 0.1% (w/v) Triton x-100) and
b) 5.3 ml stock solution of the substrate (250 mM p-nitrophenyl caprylaat (pnp) in acetonitrile). Pnp was obtained from Fluka, Zwijndrecht, Holland.

The final concentration of pnp in the substrate solution is thus 2.66 mM pnp. Subsequently, the substrate solution was placed in an ultrasonic bath for exactly 3 minutes.
Lipase solution was prepared by solubilising 3 mg/ml porcine pancreas lipase (17.7 U/gram; ICN Biomedicals BV, Zoetenneer, Holland. in buffer A
Samples were prepared by adding 40 mg of commercial extracts of Salvia material to 4 ml DMSO, incubating at room temperature for 15 minutes, followed by centrifugation to remove the insolubles. Subsequently a series of dilutions is prepared from each of the resulting solutions. For each sample the capacity to inhibit lipase activity was determined. Table 1 gives the concentration of the Salvia material or Orlistat, which gives a 50% inhibition of lipase activity.

*Lipase assay*

25 µl of sample (25 µl DMSO for control) was added to 150µl substrate solution and incubated for 10 minutes at 37 °C. Subsequently 25 µl lipase solution was added and the p-nitrophenol formation was determined during a period of 10 minutes by measuring the absorption increase at 405 nm.
Lipase inhibition was calculated by the following formula:

$$\%\ \text{Inhibition lipase activity} = (1-(\text{Act}_{\text{sample}} / \text{Act}_{\text{control}})) \times 100\%$$

wherein:

$Act_{sample}$= increase of the absorption at 405 nm in sample containing substrate, lipase and lipase inhibitor / time
$Act_{control}$ = increase of the absorption at 405 nm in sample containing substrate and lipase (control) / time

*Positive control*

As a positive control, the lipase inhibitory effect of Orlistat (Xenital®, Hoffmann-La Roche, Grenzach-Wyhlen, Germany) was determined following the above mentioned protocol for the lipase assay using a solution of 4 mg/ml or Orlistat in methanol which was further diluted with DMSO to a concentration of 0.016 mg/ml as sample.

*Results:*

| Lipase inhibitor | SA component in extract (wt.%) | Final concentration (mg/ml)[#] | Inhibition (%) |
|---|---|---|---|
| control | 0 | - | 0 |
| Salvia Miltiorrhiza extract [#1] | 6 | 1.25 | 50 |
| Salvia Miltiorrhiza extract #2 | > 60 | 0.125 | 50 |
| Orlistat [#3] | 0 | 0.002 | 50 |

[#] Amount of plant material or amount of Orlistat used per ml of test solution
[#1] Draco Natural Products Inc., 539 Parrott Street, San Jose, CA 95112, USA
[#2] TSI (Technical Sourcing International), Missoula, MT, USA
[#3] Xenital®, Hoffmann-La Roche, Grenzach-Wyhlen, Germany

## Claims

1. Use of salvianolic acid component in the manufacture of a composition for use in a method for the treatment or prevention of overweight and related disorders in a mammal, said method comprising enterally administering to said mammal an effective amount of salvianolic acid component, a pharmaceutically acceptable salt or a precursor thereof,
wherein the salvianolic acid component has the formula

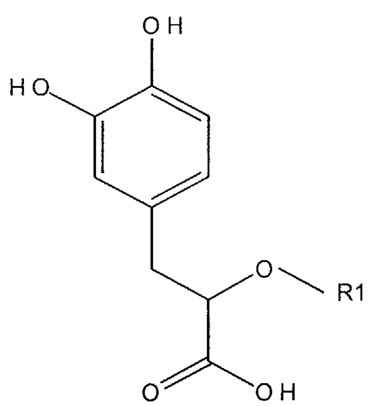

in which R1 is selected from $C_{1-50}$ hydrocarbyl, $C_{1-50}$ substituted hydrocarbyl, $C_{1-50}$ heterohydrocarbyl, $C_{1-50}$ substituted heterohydrocarbyl.

2. Use as claimed in claim 1, wherein R1 represents (CO)-R2 in which R2 is selected from optionally substituted alkyl, alkenyl, alkynyl, cycloalkyl, heterocyclyl, aryl and heteroaryl.

3. Use as claimed in claim 2, wherein R2 represents CH=CH-R3, in which R3 is selected from optionally substituted alkyl, alkenyl, alkynyl, cycloalkyl, heterocyclyl, aryl and heteroaryl.

**4.** Use according to claim 3, wherein R3 has the formula

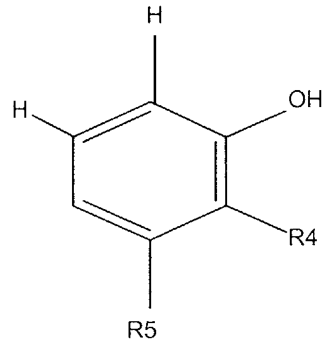

in which:

(i) R4 represents hydroxyl; R5 is selected from optionally substituted alkyl, alkenyl, alkynyl, cycloalkyl, heterocyclyl, aryl and heteroaryl; or
(ii) R4 and R5 together form an optionally substituted tetrahydrofuran radical in which R4 represents O-. .

**5.** Use according to any of one of the preceding claims, wherein the salvianolic acid component comprises at least two dihydroxyphenyl moieties, preferably at least three dihydroxyphenyl moieties.

**6.** Use according to claim 5, wherein the salvianolic acid component has any one of the formulas:

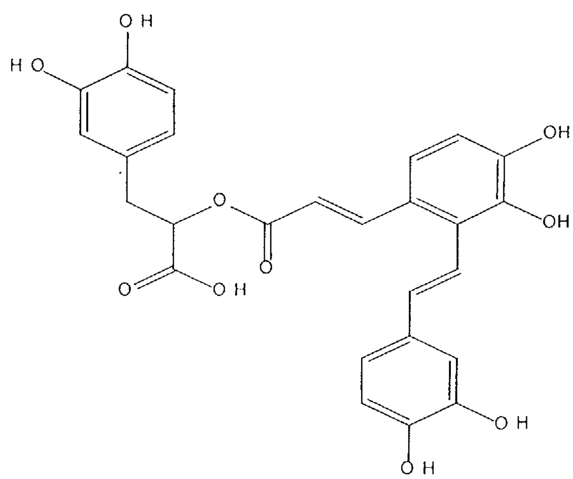

or

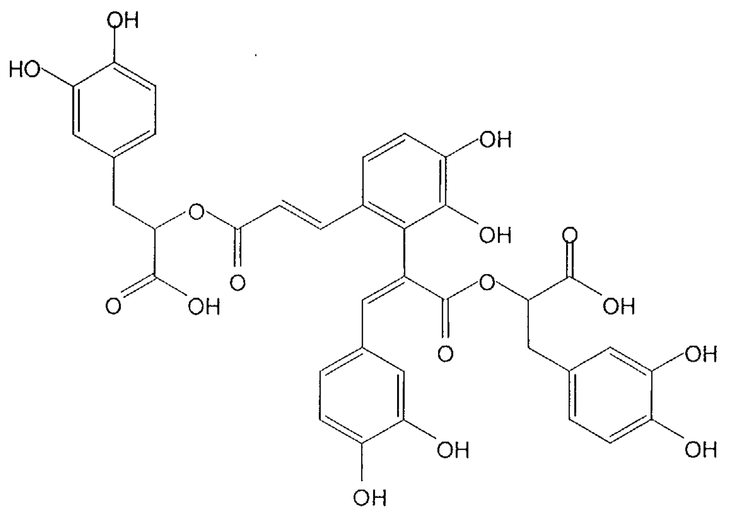

or

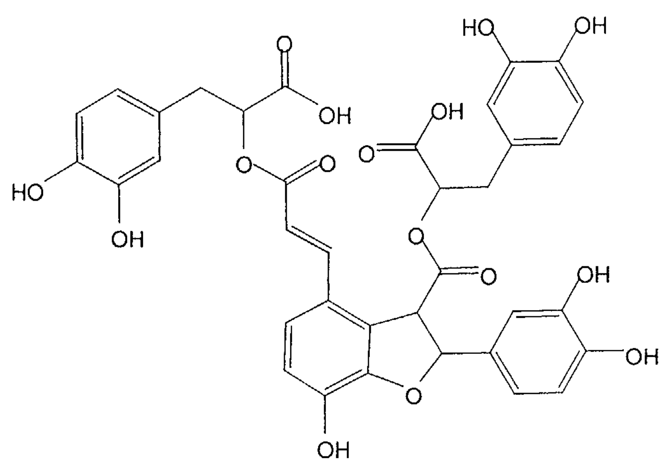

or

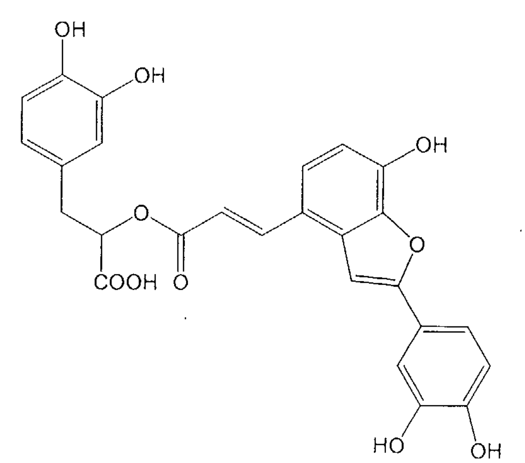

**7.** Use according to any one of the preceding claims, wherein the salvianolic acid component is plant derived.

**8.** Use according to any one of the preceding claims, wherein the method comprises the administration of a plant isolate which contains at least 5 wt.% salvianolic acid component based on the dry weight of the isolate, preferably at least 10 wt.% salvianolic acid component based on the dry weight of plant isolate.

**9.** Use according to claim 8, wherein the plant isolate is obtained from a plant belonging to the genus Salvia.

**10.** Use according to claim 9, wherein the plant isolate is obtained from *Salvia miltiorrhiza.*

**11.** Use according to any one of the preceding claims, comprising the administration of between 0.1 to and 250 mg salvianolic acid component per kg of bodyweight on a daily basis, preferably between 0.5 and 100 mg salvianolic acid component per kg of body weight on a daily basis.

**12.** Use according to any one of the preceding claims, in a method for the prevention and/or reduction of intestinal fat absorption.

**13.** Use according to claim 12, in a method for the reduction of gastrointestinal lipase activity.

**14.** Use according to any one of the preceding claims, in a method for the reduction of adipose tissue mass.

**15.** Use according to any one of the preceding claims, wherein the method further comprises the administration of a fat-soluble vitamin.

**16.** Use of salvianolic acid component as defined in claim 1 as a lipase inhibitor.

**17.** A composition suitable for the treatment of overweight comprising:

a. between 25 mg and 10 g salvianolic acid component as defined in claim 1 and
b. between 50 mg and 50 g chitosan and/or plant fiber capable of reducing fatty stool.

**18.** A composition suitable for the treatment of overweight comprising:

a. between 25 mg and 10 g salvianolic acid component as defined in claim land
b. between 10% and 250% of the RDA of one or more of a fat soluble vitamins

**19.** A food product containing between 10 and 95 wt.% triglycerides based on the total weight of the food product and between 0.01 mg and 100 mg SA component per gram of triglycerides.

European Patent Office

# EUROPEAN SEARCH REPORT

Application Number
EP 02 07 7271

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE DDFU [Online]<br>HU Y Y ET AL: "Actions of salvianolic acid A on CCl4-poisoned liver injury and fibrosis in rats. (Chinese)."<br>retrieved from STN<br>Database accession no. 1998-00133<br>XP002219621<br>* abstract *<br>& ACTA PHARMACOL.SIN. (18, NO. 5, 478-80, 1997) 1 TAB. 9 REF. CODEN: CYLPDN ISSN: 0253-9756,<br><br>Univ.Shanghai-Trad.Chinese-Med.;Inst.Materia-Medica-Shanghai; Chinese-Acad.Sci.<br>--- | 1-7, 12-14 | A61K31/216<br>A61K31/192<br>A61K31/343<br>A61K35/78<br>A61P3/04 |
| X | COOLE REZEPTE, [Online]<br>2 February 2000 (2000-02-02), XP002219769<br>Retrieved from the Internet:<br><URL:http://www.mysunrise.ch/users/h.paulweber/rezepte/salbeibutter.html><br>[retrieved on 2002-11-06]<br>whole document<br>--- | 19 | |
| A | WU Y-J ET AL: "INCREASE OF VITAMIN E CONTENT IN LDL AND REDUCTION OF ATHEROSCLEROSIS IN CHOLESTEROL-FED RABBITS BY A WATER-SOLUBLE ANTIOXIDANT-RICH FRACTION OF SALVIA MILTIORRHIZA"<br>ARTERIOSCLEROSIS, THROMBOSIS, AND VASCULAR BIOLOGY, XX, XX,<br>vol. 18, no. 3, 1998, pages 481-486,<br>XP001051813<br>ISSN: 1079-5642<br>Abstract; p. 483, right hand col., 3. paragraph (Animals)<br>---<br>-/-- | 1-14 | TECHNICAL FIELDS SEARCHED (Int.Cl.7)<br>A61K<br>A61P |

The present search report has been drawn up for all claims

| Place of search | Date of completion of the search | Examiner |
|---|---|---|
| MUNICH | 8 November 2002 | Büttner, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 07 7271

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | PATENT ABSTRACTS OF JAPAN<br>vol. 013, no. 302 (C-616),<br>12 July 1989 (1989-07-12)<br>& JP 01 090131 A (SHISEIDO CO LTD),<br>6 April 1989 (1989-04-06)<br>* abstract *<br>--- | 1-19 | |
| A | DATABASE WPI<br>Section Ch, Week 199932<br>Derwent Publications Ltd., London, GB;<br>Class B04, AN 1999-371661<br>XP002219622<br>& CN 1 211 424 A (WANG J),<br>24 March 1999 (1999-03-24)<br>* abstract *<br>----- | 1-19 | |

TECHNICAL FIELDS SEARCHED (Int.Cl.7)

The present search report has been drawn up for all claims

| Place of search | Date of completion of the search | Examiner |
|---|---|---|
| MUNICH | 8 November 2002 | Büttner, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 07 7271

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2002

| Patent document cited in search report | | Publication date | Patent family member(s) | Publication date |
|---|---|---|---|---|
| JP 01090131 | A | 06-04-1989 | NONE | |
| CN 1211424 | A | 24-03-1999 | NONE | |

EPO FORM P0459

For more details about this annex : see Official Journal of the European Patent Office, No. 12/82